# FASCICULE DE BREVET EUROPEEN

(11) **EP 0 658 350 B1**
(45) Date de publication et mention de la délivrance du brevet: **29.08.2001**
(21) Numéro de dépôt: 94402914.9
(22) Date de dépôt: 16.12.1994
(51) Int. Cl.: A61L 15/16, A61L 15/18, A61G 9/00

(54) **Composition absorbante et désinfectante et dispositif la contenant**
Absorbierendes und desinfizierendes Mittel und dieses enthaltende Vorrichtung
Absorbant and disinfectant composition and device containing same

(30) Priorité: 16.12.1993 FR 9315191
(43) Date de publication de la demande: 21.06.1995
(73) Titulaire: Wackermann, Guy, F-67100 Strasbourg (FR); Wackermann, Pierre, F-67201 Eckbolsheim (FR)
(72) Inventeur: Wackermann, Guy, F-67100 Strasbourg (FR); Wackermann, Pierre, F-67201 Eckbolsheim (FR)
(74) Mandataire: Gallochat, Alain

(56) Documents cités:
- EP-A- 0 138 427
- EP-A- 0 520 865
- WO-A-91/16877
- FR-A- 1 519 009
- FR-A- 2 706 773
- GB-A- 2 259 858
- US-A- 3 713 178
- US-A- 5 005 520
- S. BLOCK 'Disinfection, Sterilization, and Preservation' 1991 , LEA & FEBIGER , PHILADELPHIA, US 190810 * page 313, colonne de gauche, dernier alinéa - page 314, colonne de droite, alinéa 3 *
- DATABASE WPI Section Ch, Week 0887 Derwent Publications Ltd., London, GB; Class A97, AN 87-054510 & JP-A-62 011 528 (TOPPAN PRINTING KK) , 20 Janvier 1987

## Description

La présente invention concerne une nouvelle composition absorbante et désinfectante et un dispositif la contenant, utilisable pour la récupération des urines et des fèces.

La présente invention trouve plus particulièrement son application pour les malades, blessés ou personnes âgées qui sont alités.

Pour ces patients, le personnel soignant doit normalement apporter le pot à selles, ou disposer une alèse absorbante, puis vider le pot, remettre une nouvelle alèse et, cas échéant, toiletter le patient ; ces diverses opérations sont contraignantes, parfois difficilement supportables physiquement et psychologiquement, y compris pour le patient.

En outre, le risque de contamination du personnel soignant est loin d'être négligeable, en raison des nombreuses bactéries, dont certaines ont un pouvoir pathogène important, que l'on peut trouver dans les fèces.

La composition absorbante et désinfectante selon l'invention, et le dispositif la contenant permet de pallier ces principaux inconvénients et offre un confort accru pour le patient et le personnel soignant, ainsi qu'une diminution du risque de contamination.

De façon plus précise, la composition absorbante et désinfectante selon la présente invention comprend au moins deux composés se présentant sous forme de poudre ou de granulés, dont l'un a essentiellement une fonction absorbante et l'autre a un pouvoir désinfectant grâce à l'hydrolyse qui se produit lorsqu'il entre en contact avec les urines ou les fèces.

Selon un mode préférentiel de l'invention, le composé absorbant est du polyacrylate de sodium et le composé désinfectant est du perborate de sodium.

Selon un autre mode préférentiel de l'invention, le composé absorbant précité est remplacé en tout ou partie par de la sciure.

A titre préféré, la composition précitée sera répartie dans une enveloppe en matériau non tissé, elle-même disposée dans une boîte de faible épaisseur, en carton, et munie d'une ouverture disposée sous le patient.

Selon un mode particulier de l'invention, la boîte où se trouve l'enveloppe contenant la composition sera dotée d'un couvercle permettant une meilleure manipulation pour retirer, jeter et détruire la boîte après usage.

La présente invention sera mieux comprise à la lecture de la description qui va suivre accompagnée des figures parmi lesquelles :
- la figure 1 représente un exemple d'enveloppe contenant les composés absorbant et désinfectant,
- la figure 2 illustre une présentation de ladite enveloppe,
- la figure 3 illustre une variante de l'enveloppe représentée à la figure 2,
- la figure 4 représente un modèle de découpe pour la boîte contenant ladite enveloppe, et,
- la figure 5 est un exemple schématisé de dispositif selon l'invention.

Selon l'invention, la composition absorbante et désinfectante comprend au moins deux composés ; l'un d'eux est essentiellement très absorbant et se présente sous forme de granulés ou de poudre. A titre d'exemple non limitatif, ce composé peut être du polyacrylate de sodium (du type SANWET IM 5000 S vendu par la Société HOECHST) ; il présente une très bonne capacité d'absorption des liquides, tout en étant chimiquement neutre. Ce composé peut être remplacé en tout ou partie par une substance végétale du type sciure préalablement déshydratée et stérilisée ; cette substance présente également d'excellentes qualités d'absorption et est aussi chimiquement neutre. En outre, ce bio-absorbant bénéficiera de par son traitement préalable d'un puissant pouvoir bactériostatique.

S'agissant du composé désinfectant, qui se présente également sous forme de poudre ou de granulés, la préférence a été donnée au perborate de sodium dont les qualités oxydantes permettent une action bactéricide satisfaisante, évitant ainsi toute propagation de germes, notamment pathogènes, lorsque l'urine ou les fèces entrant en contact avec le perborate de sodium produisent son hydrolyse, libérant ainsi l'activité oxydante de ce composé ; il a été vérifié expérimentalement que, après hydrolyse, le " pH résiduel " était aux environs de 9, d'où, par voie de conséquence, une propriété bactériostatique par alcalinité.

L'activité du perborate de sodium sera renforcée à basse température par du TAED (Tétra Acétyl Ethylène Diamine) lui conférant en plus une activité bactéricide complémentaire. On pourra également ajouter de l'undécylénate de méthyle qui conférera au mélange une propriété bactéricide supplémentaire sous forme gazeuse aérosol, destinée à la destruction des germes libres nosocomiaux.

Préférentiellement, ces composés seront dosés séparément ou mélangés au choix et disposés dans des poches réalisées en matériau non tissé ; ce matériau pourra préalablement être enduit sur une face d'un produit thermocollant par points. Il sera alors aisé par simple soudure (le produit thermocollant présentant par exemple une température de fusion de l'ordre de 130-140° C), de réaliser des enveloppes à partir dudit matériau, chaque enveloppe pouvant elle-même se subdiviser en plusieurs poches contenant les divers composés précités. Il est également possible d'envisager des coussinets à compartiments multiples.

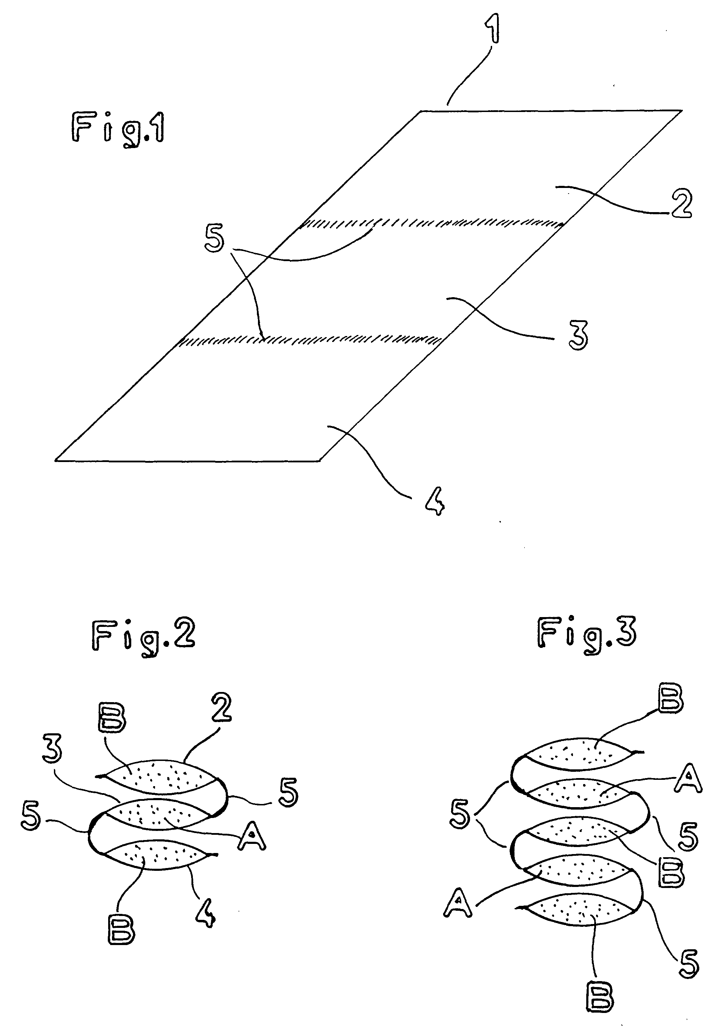

La figure 1 illustre schématiquement un mode de réalisation de l'enveloppe 1 réalisée en matériau non tissé, comportant trois poches 2, 3 et 4 séparées par des lignes de soudure 5 ; le composé absorbant pourra se trouver dans la poche 3, alors que le matériau désinfectant se situera dans les poches 2 et 4, ces dernières étant repliées respectivement au dessus et en dessous de la poche 3 le long des lignes de soudure 5. Dans cette disposition, illustrée à la figure 2, le composé absorbant A sera donc pris en sandwich par le composé désinfectant B.

Selon la figure 3, l'enveloppe 1 comportera cinq poches séparées par des lignes de soudure et contenant en alternance du composé A et du composé B, ainsi que cela est représenté, de telle sorte qu'après repliement, le composé absorbant A sera là encore pris en sandwich par le composé désinfectant B.

D'autres configurations sont bien entendu possibles au niveau de l'enveloppe et des différentes poches contenant les composés absorbant et désinfectant, mais la préférence ira vers une enveloppe constituée d'un chapelet de boudins longilignes juxtaposés le long de leur longueur et remplis alternativement de composés A et B, ainsi que cela a été décrit précédemment.

A titre purement indicatif, chaque enveloppe pourra contenir dans les conditions précitées, de 0,4 à 1,41 de perborate de sodium et de 1,5 à 21 de bio-absorbant, les dimensions de l'enveloppe dépliée variant de 1000 à 1200mm sur 350mm environs.

L'utilisateur recevra donc ces enveloppes fermées contenant déjà les composés A et B ; il recevra également une découpe, par exemple en carton, qui, une fois mise en forme aboutira à une boîte parallélépipédique, de faible épaisseur dans laquelle sera disposée l'enveloppe précitée.

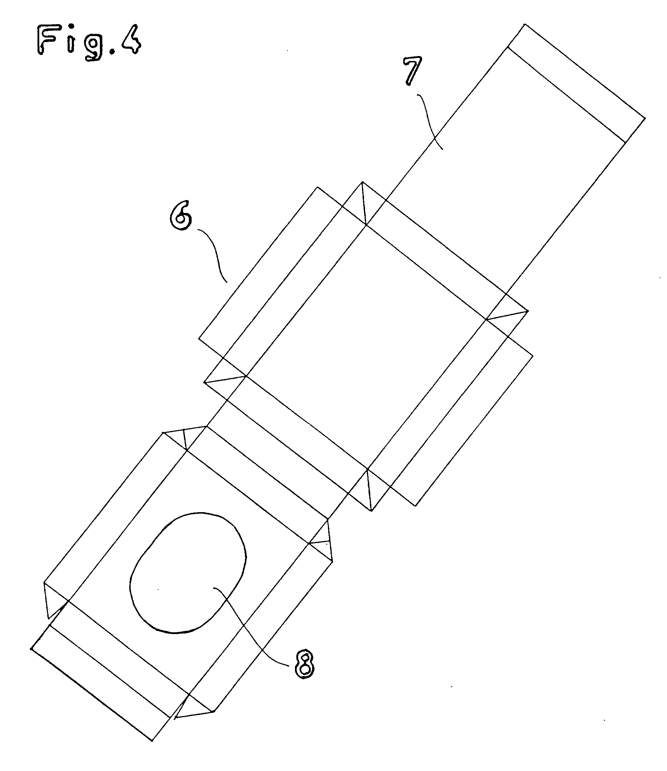

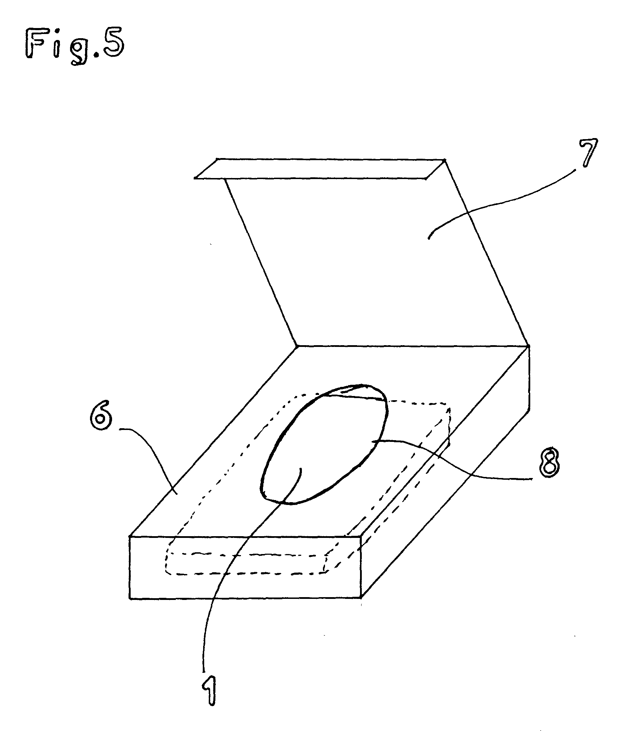

La figure 4 illustre une telle découpe qui, une fois mise en forme grâce aux pattes et rabats préférentiellement préencollés, donnera une boîte 6 illustrée à la figure 5, où aura été préalablement disposée l'enveloppe 1 elle-même, c'est-à-dire les poches, par exemple les boudins longilignes, déjà remplies de composés A et B ; le couvercle 7 permet, après utilisation, de retirer la boîte et de la détruire en toute sécurité et dans des conditions d'hygiène renforcée.

Dans la pratique, l'utilisateur mettre en forme la boîte 6, disposera le couvercle 7 sous la boîte 6, et placera cette dernière sous le patient en disposant convenablement l'ouverture 8 contre ce dernier, permettant aux urines et aux fèces de se répartir sur l'enveloppe 1 où les composés A et B joueront leur rôle d'absorbant et de désinfectant. Un support plastique, non représenté ici pourra servir de réceptacle pour disposer la boîte et son contenu.

Diverses variantes sont possibles au niveau de la boîte ; en particulier, le carton pourra avantageusement être imperméabilisé. En outre et afin d'augmenter la rigidité de l'ensemble, des renforts internes pourront être prévus à l'intérieur de ladite boîte.

Ce dispositif permet en conséquence une amélioration substantielle des conditions de travail et d'hygiène pour le personnel soignant des établissements hospitaliers ou de repos, voire pour des particuliers ayant à leur charge des personnes alitées.

## Revendications

1. Composition absorbante et désinfectante, à base de matériaux pulvérulents ou sous forme de granulés, destinée à recevoir les urines et les fèces de personnes, notamment alitées, âgées et/ou malades, **caractérisée en ce qu**'elle présente un pH résiduel alcalin de l'ordre de 9 après hydrolyse des dites urines et fèces par lesdits matériaux.

2. Composition selon la revendication 1 **caractérisée en ce qu**'elle comprend de 0,4 à 1,4 1 de composé désinfectant et de 1,5 à 2 1 de composé absorbant.

3. Composition selon l'une des revendications 1 ou 2 **caractérisée en ce que** le composé désinfectant comprend du perborate de sodium.

4. Composition selon la revendication 3 **caractérisée en ce que** le composé désinfectant comprend en outre du Tétra Acétyl Ethylène Diamine ou de l'undécylénate de méthyle.

5. Composition selon l'une quelconque des revendications 1 à 4 **caractérisée en ce que** le matériau absorbant est de la sciure de bois.

6. Composition selon d'une quelconque des revendications 1 à 5 **caractérisée en ce que** chacun desdits composés se trouve dans une poche en matériau non tissé.

7. Composition selon la revendication 6 **caractérisée en ce que** chacune desdites poches se présente sous forme d'un boudin longiligne relié au précédent le long de sa longueur, l'ensemble desdites poches constituant une enveloppe.

8. Composition selon la revendication 7 **caractérisée en ce que** chaque poche contenant le composé absorbant est prise en sandwich par une poche contenant le composé désinfectant.

## Patentansprüche

1. Absorbierendes und desinfizierendes Gemisch auf der Basis pulverförmiger oder körniger Stoffe, das dazu bestimmt ist, Urin und Stuhl insbesondere bettlägeriger alter und/oder kranker Menschen aufzunehmen, **dadurch gekennzeichnet**, daß es nach erfolgter Hydrolyse des Urins und des Stuhls durch die besagten Stoffe einen restlichen basischen pH-Wert von etwa 9 aufweist.

2. Gemisch nach Anspruch 1, **dadurch gekennzeichnet** daß es zwischen 0,4 und 1,4 l desinfizierenden Stoff und zwischen 1,5 und 2 l absorbierenden Stoff aufweist.

3. Gemisch nach Anspruch 1 oder 2, **dadurch gekennzeichnet** daß der desinfizierende Stoff Natriumperborat enthält

4. Gemisch nach Anspruch 3, **dadurch gekennzeichnet** daß der desinfizierende Stoff zusätzlich Tetraazethyläthylendiamin oder Methylundecylenat enthält.

5. Gemisch nach einem der Ansprüche 1 bis 4, **dadurch gekennzeichnet** daß der absorbierende Stoff Sägemehl ist

6. Gemisch nach einem der Ansprüche 1 bis 5, **dadurch gekennzeichnet** daß jeder der genannten Stoffe in einem Beutel aus nicht gewebtem Material enthalten ist.

7. Gemisch nach Anspruch 6, **dadurch gekennzeichnet** daß jeder der genannten Beutel als langgestreckter, langsseitig mit dem vorangehenden verbundener Sack ausgebildet ist, wobei die Gesamtheit der Beutel eine Hülle bildet.

8. Gemisch nach Anspruch 7, **dadurch gekennzeichnet** daß jeder den absorbierenden Stoff enthaltende Beutel von einem den desinfizierenden Stoff enthaltenden Beutel umgeben ist.

## Claims

1. An absorbing and disinfecting composition, comprising pulverulent or granulated material, aimed to receive urines and faeces from people, specially being bedridden, elderly and/or unhealthy, whereas said composition has a residual alkaline pH of around 9 after the hydrolysis of said urines and faeces by said material.

2. A composition according to claim 1 whereas it comprises from 0,4 to 1,4 1 of disinfectant agent, and from 1,5 to 2 1 of absorbing agent.

3. A composition according to any of claims 1 or 2 whereas the disinfectant agent comprises sodium perborate.

4. A composition according to claim 3 whereas the disinfectant agent further comprises TAED or methylundecylenate.

5. A composition according to any of claims 1 to 4 whereas the absorbing agent is sawdust.

6. A composition according to any of claims 1 to 5 whereas each of the agents is contained in a bag made of non-woven material.

7. A composition according to claim 6 whereas each bag is roughly formed as a cylinder lengthwise connected to other cylinders, all said bags forming an envelope.

8. A composition according to claim 7 whereas each bag containing the absorbing agent is surrounded by bags containing the disinfectant agent.
